# FASCICULE DE BREVET EUROPEEN

(11) **EP 3 149 445 B1**
(45) Date de publication et mention de la délivrance du brevet: **07.10.2020**
(21) Numéro de dépôt: 15734447.4
(22) Date de dépôt: 27.05.2015
(51) Int. Cl.: G01N 1/28, C12N 1/06, C12M 1/00

(54) **DISPOSITIF DE LYSE D'ESPECES BIOLOGIQUES ET PROCEDE MIS EN OEUVRE PAR CE DISPOSITIF**
VORRICHTUNG ZUR LYSIERUNG BIOLOGISCHER SPEZIES UND DURCH DIE BESAGTE VORRICHTUNG IMPLEMENTIERTES VERFAHREN
DEVICE FOR LYSING BIOLOGICAL SPECIES AND METHOD IMPLEMENTED BY SAID DEVICE

(30) Priorité: 28.05.2014 FR 1454866
(43) Date de publication de la demande: 05.04.2017
(73) Titulaire: COMMISSARIAT À L'ÉNERGIE ATOMIQUE ET AUX ÉNERGIES ALTERNATIVES, 75015 Paris (FR)
(72) Inventeur: BOURDAT, Anne-Gaelle, F-38260 Nantoin (FR); VIANA, Antonio, F-38320 Herbeys (FR); DEN DULK, Remco, F-38120 Saint-Egrève (FR)
(74) Mandataire: Gevers & Orès
(86) Numéro de dépôt international: PCT/IB2015/053970
(87) Numéro de publication internationale: WO 2015/181743

(56) Documents cités:
- WO-A1-99/02958
- US-A- 3 941 317
- US-A- 5 114 858

## Description

L'invention se rapporte à un dispositif de lyse d'espèces biologiques (micro-organismes, bactéries, cellules, spores, etc.) et à un procédé mis en œuvre par ce dispositif.

La lyse de cellules biologique est nécessaire pour récupérer et étudier le matériel intracellulaire, Par exemple, la lyse cellulaire est nécessaire pour récupérer l'ADN de la cellule en vue d'une réplication et d'une analyse, telle qu'une comparaison d'ADN.

Les méthodes classiquement utilisées pour la lyse d'espèces biologiques sont les suivantes
- La lyse chimique : cette technique comprend la mise au contact des cellules avec une solution de lyse, contenant généralement un détergent faisant éclater les cellules. Cette technique est efficace mais peut être dangereuse pour certains matériels cellulaires. En outre, elle est longue et nécessite de nombreuses manipulations, dont une étape de purification de la solution de lyse, et éventuellement de concentration du lysat, ce qui la rend longue, avec un risque de perdre une grande quantité de matériel biologique lors de la purification et de la concentration et nécessite l'intervention d'une personne spécialisée cette technique n'est pas efficace pour la lyse des spores.
- La lyse par choc thermique : cette technique comprend par exemple l'incubation de l'extrait à une température inférieure à 0°C, suivie immédiatement de l'incubation de l'extrait à une température d'au moins 95°C, de préférence environ 100°C. Cette technique nécessite un matériel complexe, notamment si on souhaite lyser des spores.
- La lyse par champ électrique : cette technique comprend l'utilisation d'une différence de potentiel conduisant à la rupture de la membrane cellulaire. Cette technique nécessite un matériel complexe et est à l'instar des méthodes précédentes, peu adaptée à la lyse de spores.
- La lyse mécanique : cette technique est basée sur l'agitation de l'extrait en présence de billes afin que les chocs des billes contre les cellules éclatent ces dernières. Cette technique nécessite un matériel complexe. En outre, il est nécessaire d'utiliser des billes parfaitement dépourvues de micro-organismes, et de les récupérer après la lyse. Des exemples de dispositifs mettant en œuvre ce procédé sont les broyeurs d'échantillons biologiques de la gamme Precellys© de la société Bertin technologies. Il s'agit de dispositifs chers et encombrants, difficilement intégrables dans un instrument compact.
- La lyse par pression : cette technique comprend l'application d'une pression hydrostatique sur les cellules, conduisant à la rupture de leur membrane. Cette technique nécessite un matériel complexe.

Parmi les procédés de lyse par broyage mécanique, on peut citer les documents suivants :
- FR 2 781 500, qui décrit un procédé de lyse mécanique de micro-organismes par mise en mouvement des moyens de broyage par un champ magnétique ; et
- US 2003/0066915, qui décrit un procédé de lyse mécanique de micro-organismes par mise en mouvement des moyens de broyage par des ultrasons.

Les documents WO-A1-99/02958 et US-A-5 114 858 divulguent chacun un dispositif de lyse mécanique d'espèces biologiques comprenant une première et une seconde parois mobiles l'une par rapport à l'autre, entre une position initiale où elles sont écartées l'une de l'autre et une position de lyse où la première paroi formant piston est en appui contre la seconde paroi, les première et seconde parois étant montées mobiles en cisaillement l'une par rapport à l'autre dans la position de lyse. Les pistons présentés dans ces deux documents sont pourvus de saillies macroscopiques qui forment une denture de broyage et ne confèrent donc pas une nicrorugosité aux surfaces d'appui correspondantes de ces pistons.

Toutes ces techniques sont complexes et nécessitent un matériel important. En outre, elles nécessitent un temps de préparation important, et une durée de lyse de plusieurs dizaines de secondes à plusieurs minutes. De plus, elles sont difficilement intégrables sur des instruments portables et compacts.

L'objectif de la présente invention est donc de proposer un appareil et un procédé de lyse d'espèces biologiques simple, peu onéreux, et facilement intégrable dans un équipement de laboratoire ou dans un dispositif portable, et présentant une efficacité de lyse élevée, en particulier sur des spores permettant une lyse satisfaisante (au moins 50 % des espèces biologiques lysées) en moins de 10 secondes, voire même en 1 seconde.

Pour cela, l'invention propose de réaliser un appareil et un procédé de lyse mécanique mettant en œuvre une surface rugueuse contre laquelle les objets à lyser sont broyés par un mouvement de friction (cisaillement) et non par des chocs ou une simple pression.

A cette fin, l'invention a pour objet un dispositif de lyse mécanique d'espèces biologiques présentes dans un fluide comprenant une première paroi et une seconde paroi montées mobiles l'une par rapport à l'autre, entre une position initiale dans laquelle les première et seconde parois sont écartées l'une de l'autre, et une position de lyse dans laquelle la première paroi est en appui contre la seconde paroi, les première et seconde parois étant également montées mobiles en cisaillement l'une par rapport à l'autre dans la position de lyse, caractérisé en ce que l'une au moins des première et seconde parois a une surface d'appui rugueuse contre l'autre paroi et présente un paramètre de rugosité de surface Ra moyen compris entre 0,2 µm et 10 µm, et préférentiellement entre 0,2 µm et 3 µm.

Selon d'autres modes de réalisation du dispositif selon l'invention :
(i) l'une des première paroi et seconde paroi peut être une paroi microporeuse, c'est-à-dire poreuse aux liquides et non poreuse aux espèces biologiques devant être lysées ;
(ii) la paroi microporeuse peut présenter un diamètre moyen de pores inférieur à 1 µm, voire inférieur à 0,2 µm ;
(iii) la paroi microporeuse peut être rugueuse ;
(iv) la paroi microporeuse rugueuse peut être constituée d'une couche de matériau microporeux ayant une surface plane sur laquelle est positionnée une couche rugueuse destinée à être en appui contre l'autre paroi en position de lyse :
(v) selon une variante de (iv), la paroi microporeuse rugueuse peut être constituée d'une couche de matériau microporeux ayant une surface rugueuse ;
(vi) selon une autre variante de (iv), ia paroi microporeuse rugueuse peut être constituée d'une couche de matériau macroporeux ayant une surface rugueuse sur laquelle est positionnée une couche microporeuse destinée à être en appui contre l'autre paroi en position de lyse, la couche microporeuse étant suffisamment souple et fine pour épouser la rugosité de la surface de la couche de matériau macroporeux en position de lyse :
(vii) la paroi microporeuse peut être non rugueuse et présenter un paramètre de rugosité de surface Ra moyen inférieur à 0,1 µm, alors que la paroi non microporeuse peut être rugueuse et présenter un paramètre de rugosité de surface Ra moyen compris entre 0,2 µm et 10 µm, préférentiellement entre 0,2 µm et 3 µm ;
(viii) selon une variante de (vii), la première paroi et la seconde paroi peuvent être rugueuses ;
(ix) le dispositif selon l'invention peut comprendre en outre une chambre et un piston monté mobile dans la chambre entre la position initiale et la position de lyse, la première paroi étant montée sur le piston mobile et la seconde paroi étant agencée en regard de la première paroi ;
(x) la seconde paroi du dispositif peut être alors microporeuse, et la chambre peut comporter en outre une sortie fluidique agencée à l'opposé de la première paroi par rapport à la seconde paroi microporeuse ;
(xi) la chambre peut alors comporter en outre une entrée fluidique et une autre sortie fluidique ; et
(xii) la chambre peut alors comporter en outre une membrane étanche agencée entre la première paroi, l'entrée fluidique et la sortie fluidique, la membrane étant suffisamment fine et souple pour épouser la rugosité de l'une au moins des première et seconde parois en position de lyse.

L'invention se rapporte également à un procédé de lyse mécanique d'espèces biologiques comprenant les étapes suivantes :
A) Fourniture d'un dispositif tel que celui précité de telle sorte que les première et seconde parois soient en position initiale ;
B) Placer les espèces biologiques à lyser entre ces parois ;
C) Amener ces parois en position de lyse :
D) Générer un cisaillement entre ces parois en position de lyse pour provoquer la lyse mécanique de ces espèces et obtenir un lysat ;
E) Écarter ces parois jusqu'à ce qu'elles soient en position initiale ; et
F) Récupérer le lysat des espèces biologiques.

Le procédé selon l'invention est efficace puisqu'il permet un rendement au moins équivalent à la méthode de référence de lyse des spores par les broyeurs Precellys© (Bertin).

Le procédé est rapide en regard de la méthode de référence (broyeur Precellys© (Bertin)) : en moins de 10 secondes contre 30 secondes pour le broyeur de référence.

Le procédé selon l'invention est économique et direct car il ne requiert pas d'ajout de substance chimique, ni de purification ultérieure, ni d'ajout de corps étrangers dans l'échantillon (tels que des billes). En outre, l'ADN récupéré à l'issue de la lyse par le procédé selon l'invention est directement analysable (i.e. sans étape de purification) à l'aide des techniques de biologie moléculaire classiques, telles que la « PCR » (« Polymerase Chain Reaction » en anglais) ou l'électrophorèse. Enfin, la libération s'effectue sans dégradation des acides nucléiques des espèces.

Selon d'autres modes de réalisation du procédé selon l'invention :
- l'étape A) peut consister à fournir un dispositif selon la caractéristique (ix) ci-dessus, l'étape C) à faire coulisser le piston dans la chambre jusqu'à ce que les première et seconde parois soient en position de lyse et l'étape D) à faire tourner le piston sur lui-même de telle sorte que ces parois restent en contact l'une avec l'autre ;
- l'étape A) peut consister à fournir un dispositif selon (x) ci-dessus, l'étape B) à placer un liquide comportant les espèces à lyser entre les parois, l'étape C) à faire coulisser le piston dans la chambre jusqu'à ce que le liquide ait été filtré à travers la paroi microporeuse, que seules ces espèces restent sur la paroi microporeuse et que ces parois soient en position de lyse, et l'étape D) à faire tourner le piston sur lui-même de sorte que ces parois restent en contact l'une avec l'autre ;
- l'étape A) peut consister à fournir un dispositif selon (xi) ci-dessus, l'étape B) à faire entrer un liquide comportant les espèces à lyser par rentrée fluidique, entre ces parois, l'étape C) à faire coulisser le piston dans la chambre jusqu'à ce que ces parois soient en position de lyse, l'étape D) à faire tourner le piston sur lui-même de sorte que ces parois restent en contact l'une avec l'autre et l'étape F) à faire circuler un liquide d'élution entre l'entrée et la sortie fluidique pour récupérer le lysat par cette sortie ; et
- l'étape A) peut consister à fournir un dispositif selon (xii) ci-dessus, l'étape B) à faire entrer un liquide comportant les espèces à lyser par rentrée fluidique, entre la membrane étanche et la seconde paroi, l'étape C) à faire coulisser le piston dans la chambre jusqu'à ce que ces parois soient en position de lyse par l'intermédiaire de la membrane, l'étape D) à faire tourner le piston sur lui-même de telle sorte que ces parois restent en contact l'une avec l'autre par l'intermédiaire de la membrane, et l'étape F) à faire circuler un liquide d'élution entre l'entrée fluidique et la sortie fluidique pour récupérer le lysat par la sortie fluidique.

D'autres caractéristiques de l'invention seront énoncées dans la description détaillée ci-après, faite en référence aux figures annexées qui représentent, respectivement :
- les figures 1 et 2, deux vues schématiques en coupe d'un premier mode de réalisation d'un dispositif de lyse cellulaire selon l'invention ;
- la figure 3, une vue schématique en coupe d'un deuxième mode de réalisation d'un dispositif de lyse cellulaire selon l'invention ;
- la figure 4, une vue schématique en coupe d'un troisième mode de réalisation d'un dispositif de lyse cellulaire selon l'invention ;
- la figure 5, une vue schématique en coupe d'un quatrième mode de réalisation d'un dispositif de lyse cellulaire selon l'invention ;
- la figure 6, une vue schématique en coupe d'un cinquième mode de réalisation d'un dispositif de lyse cellulaire selon l'invention ;
- les figures 7 et 8, deux vues schématiques en coupe d'un sixième mode de réalisation d'un dispositif de lyse cellulaire selon l'invention ;
- la figure 9, une vue schématique en coupe d'un septième mode de réalisation d'un dispositif de lyse cellulaire selon l'invention ;
- la figure 10, une vue schématique en coupe d'un huitième mode de réalisation d'un dispositif de lyse cellulaire selon l'invention ; et
- les figures 11 et 12, deux vues schématiques en coupe d'un neuvième mode de réalisation d'un dispositif de lyse cellulaire selon l'invention.

Dans la description, les termes suivants sont définis comme suit :
- Surface rugueuse : il s'agit d'une surface présentant un paramètre de rugosité de surface Ra moyen, correspondant à la moyenne arithmétique de mesures de paramètre Ra, supérieur à 0,2 µm, de préférence compris entre 0,2 µm et 10 µm et encore plus préférentiellement entre 0,2 µm et 3 µm, (le paramètre ou coefficient de rugosité moyen de surface Ra est établi à partir d'une mesure au profilomètre, comme détaillé par la suite) :
- Microporeux : il s'agit d'une paroi ou d'une couche de matériau comprenant des pores ouverts suffisamment larges pour laisser passer les liquides, tels que l'eau, mais suffisamment étroits pour empêcher de passer les espèces biologiques devant être lysées ainsi que le matériel biologique issu de la lyse et devant être analysé. Typiquement, une paroi microporeuse au sens de l'invention comporte des pores ouverts ayant un diamètre inférieur à 1 µm, et de préférence inférieur à 0,2 µm. A titre d'exemple, on peut citer les filtres frittés, les filtres en silice poreuse, les filtres en céramique, et les filtres en polymère poreux ou en métal poreux ;
- Macroporeux: il s'agit d'une paroi, d'une couche ou d'un matériau comprenant des pores ouverts ou des canaux suffisamment larges pour laisser passer les liquides, tels que l'eau, mais également suffisamment large pour laisser passer les espèces biologiques devant être lysées ou le matériel biologique issu de la lyse et devant être analysé. Typiquement, une paroi macroporeuse au sens de l'invention comporte des pores ouverts ou des canaux ayant un diamètre supérieur à 0,2 µm, et de préférence supérieur à 1 µm. A titre d'exemple, on peut citer une couche de verre, de métal ou de polymère munie de canaux traversant la couche ;
- Espèce biologique ; notamment des cellules, micro-organismes, en particulier bactéries, spores, virus, champignons, microalgues.

Afin de réaliser une lyse d'espèces biologiques, telles que des micro-organismes, des cellules, des spores, etc., l'invention propose un dispositif de lyse mécanique comprenant une première paroi et une seconde paroi montées mobiles l'une par rapport à l'autre, entre une position initiale dans laquelle les première et seconde parois sont écartées l'une de l'autre, et une position de lyse dans laquelle la première paroi est en appui contre la seconde paroi.

Les première et seconde parois sont également montées mobiles en cisaillement l'une par rapport à l'autre, dans la position de lyse, c'est-à-dire l'une en appui contre l'autre. Le cisaillement peut être obtenu par une rotation ou par une translation de la première paroi par rapport à la seconde paroi. L'invention prévoit donc deux degrés de liberté entre les parois : un degré de liberté pour approcher les parois l'une au contact de l'autre, et un degré de liberté pour permettre un cisaillement lorsque les parois sont en appui l'une contre l'autre.

Selon l'invention, l'une au moins des première et seconde parois a une surface rugueuse d'appui contre l'autre paroi. En d'autres termes, la surface rugueuse présente un paramètre de rugosité de surface Ra moyen, compris entre 0,2 µm et 10 µm, de préférence entre 0,2 µm et 3 µm.

A titre d'exemple de pression d'appui, des tests ont été réalisés en utilisant une lame de verre dépolie et une lame de verre classique maintenues entre le pouce et l'index d'une main. La seule force des doigts appliquée en pression et en cisaillement suffit à obtenir une lyse des espèces placées entre les deux lames.

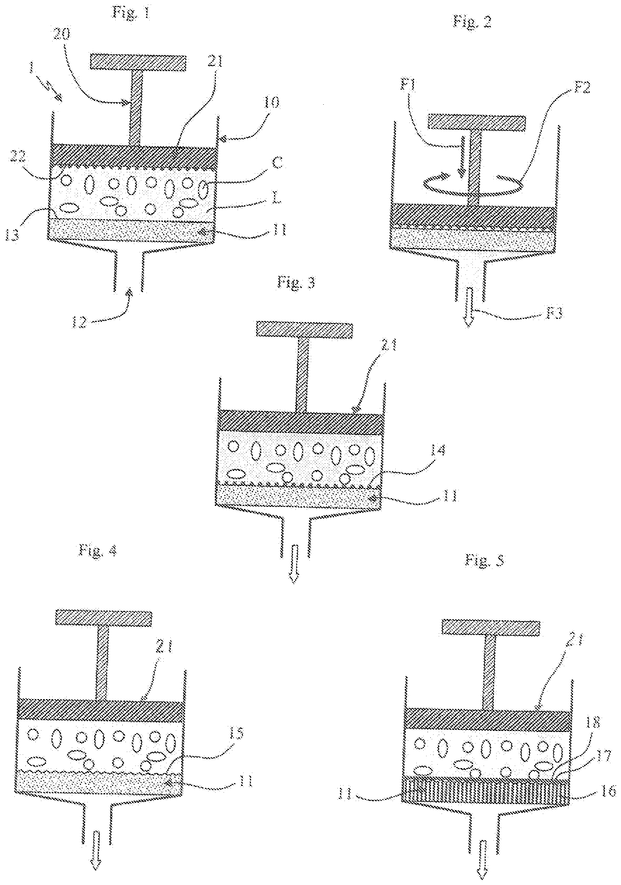

Un premier mode de réalisation d'un dispositif selon l'invention est illustré aux figures 1 et 2.

À la figure 1, le dispositif selon l'invention est en position initiale dans laquelle les première et seconde parois sont écartées l'une de l'autre.

Le dispositif de lyse mécanique 1 comprend une chambre 10 et un piston 20 monté mobile en translation dans la chambre 10 entre la position initiale (figure 1) et la position de lyse (figure 2). Un joint (non illustré) est prévu entre le piston et la chambre pour assurer l'étanchéité. La première paroi 21 est montée sur le piston mobile 20 et la seconde paroi 11 est agencée en regard de la première paroi 21.

Dans ce mode de réalisation, la seconde paroi 11 est microporeuse afin de permettre au liquide L comportant les cellules C à lyser de diffuser à travers la seconde paroi 11 lorsque le piston 20 est abaissé jusqu'à la position de lyse dans laquelle les deux parois 11 et 21 sont en contact l'une de l'autre (figure 2). La seconde paroi 11 est par exemple un filtre poreux en polymère. Avantageusement, la chambre 10 comporte, en outre, une sortie fluidique 12 agencée à l'opposé de la première paroi 21 par rapport à la seconde paroi microporeuse 11, permettant l'évacuation du liquide ainsi que des espèces lysées. Dans une alternative non illustrée, un réservoir peut remplacer la sortie fluidique, pour recevoir le liquide.

La paroi microporeuse 11 présente un diamètre moyen de pores compris entre 0,2 µm et 0,5 µm. Le diamètre des pores est adapté pour assurer que le liquide diffuse à travers la paroi 11, mais que les cellules intègres (i.e. avant la lyse) restent à la surface de la paroi à la surface de laquelle elles sont lysées. Le matériel biologique à étudier (ADN, protéines, etc.) libéré par la lyse peut passer à travers le filtre ou rester à la surface de ce dernier, pour être récupéré.

Dans le mode de réalisation illustré aux figures 1 et 2, c'est la paroi 21 portée par le piston 20 qui présente une surface d'appui rugueuse 22 contre l'autre paroi. Autrement dit, la paroi microporeuse 11 présente une surface 13 non rugueuse, alors que la paroi non microporeuse 21 présente une surface 22 rugueuse, par exemple une surface abrasive.

Alternativement, comme illustré à la figure 3, c'est la paroi microporeuse 11 contre laquelle est disposée une surface 14 rugueuse. Par exemple, la paroi microporeuse rugueuse 11 est constituée d'une couche 11 de matériau microporeux ayant une surface 13 plane sur laquelle est positionnée une couche rugueuse 14 destinée à être en appui contre la paroi 21 du piston 20 en position de lyse. Dans ce mode de réalisation, la couche rugueuse 14 doit pouvoir laisser passer le liquide dans lequel se trouvent les cellules à lyser.

A titre d'exemple, on peut utiliser une couche rugueuse 14 de 500 µm, à 2 mm d'épaisseur, et une couche microporeuse 11 de 500 µm à 5 mm d'épaisseur. La couche rugueuse 14 est constituée d'un matériau poreux abrasif, par exemple une grille métallique.

La figure 4 illustre un mode de réalisation alternatif de paroi microporeuse 11 rugueuse.

Dans ce mode de réalisation, la paroi microporeuse rugueuse 11 est constituée d'une couche de matériau microporeux ayant une surface 15 rugueuse, par exemple microstructurée. Autrement dit, la rugosité de la surface 15 est obtenue par microstructuration d'un matériau microporeux. Cette couche 11 de matériau microporeux peut également être constituée d'un filtre abrasif, tel qu'un filtre fritté, une silice poreuse, une céramique, un polymère poreux. Il s'agit par exemple d'un filtre en acier inoxydable, se présentant sous la forme d'une grille.

Quel que soit le mode de réalisation représenté sur les figures 1 à 4, la paroi microporeuse 11 permet de retenir les espèces biologiques à lyser, tout en laissant s'écouler le liquide biologique. Suite à l'opération de lyse, les espèces biologiques lysées s'écoulent à travers la paroi microporeuse 11.

La figure 5 illustre un quatrième mode de réalisation alternatif de paroi microporeuse 11 rugueuse.

Dans ce mode de réalisation, la paroi microporeuse rugueuse 11 est constituée d'une couche macroporeuse 16 ayant une surface 17 rugueuse, et d'une couche microporeuse 18, positionnée sur la couche macroporeuse 16 et destinée à être en appui contre la première paroi 21 portée par le piston 20, en position de lyse,

La couche microporeuse 18 doit être suffisamment souple et fine pour épouser la rugosité de la surface 17 de la couche macroporeuse 16 en position de lyse, afin que la rugosité de la couche macroporeuse 16 transparaisse à travers la couche microporeuse 18 et que les cellules puissent être lysées. Grâce à la couche microporeuse 18, les espèces biologiques à lyser sont retenues et peuvent être lysées.

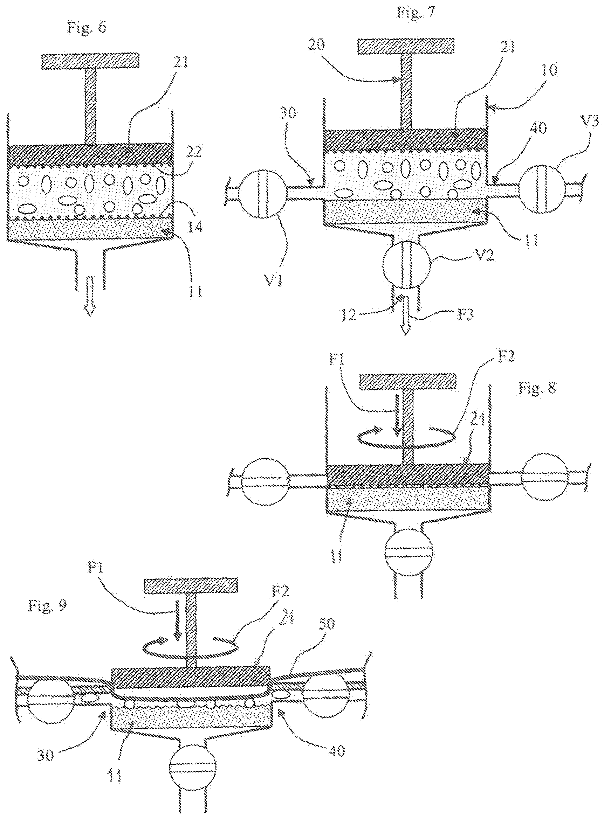

La figure 6 illustre un cinquième mode de réalisation dans lequel la première paroi 21 et la seconde paroi 11 sont toutes les deux rugueuses, c'est-à-dire qu'elles ont toutes les deux une surface 22-14 rugueuse. Elles peuvent être constituées par une combinaison des modes de réalisation des figures 1 et 3, une combinaison des modes de réalisation des figures 1 et 4 ou une combinaison des modes de réalisation des figures 1 et 5.

Les figures 7 et 8 illustrent un sixième mode de réalisation permettant une mise en œuvre facilitée de lyse et de récupération du matériel biologique à analyser.

Dans ce mode de réalisation, la chambre 10 comporte, en outre, une entrée fluidique 30 et une sortie fluidique 40 agencée, vue en coupe, entre les première et seconde parois 21-11 lorsqu'elles sont en position initiale.

En position initiale, du liquide L comportant les cellules C à lyser est introduit dans la chambre 10, entre la première paroi 21 et la seconde paroi 11, en ouvrant la vanne V1 de l'entrée fluidique 30, et en fermant les vannes V2 et V3, respectivement des sorties fluidiques 12 et 40. Cette étape permet de placer les cellules biologiques C à lyser entre les première et seconde parois 21-11.

Une fois la chambre 10 remplie de liquide, la vanne V1 est fermée, et la vanne V2 est ouverte. Le piston 20 est alors amené en position de lyse selon la flèche F1 (figure 8). Le liquide peut s'écouler à travers la paroi microporeuse 11 et être évacué selon la flèche F3 par la sortie fluidique 12 agencée à l'opposé de la première paroi 21 par rapport à fa seconde paroi microporeuse 11.

Ainsi, seules les cellules biologiques C à lyser restent sur la paroi microporeuse 11.

Une fois que le piston 20 portant la première paroi 21 est en appui contre la seconde paroi microporeuse 11 en position de lyse, le piston 20 est mis en rotation selon la flèche F2 pour générer un cisaillement entre les première et seconde parois 21 et 11 et provoquer la lyse mécanique des cellules biologiques et obtenir un lysat. Durant cette étape, représentée sur la figure 8, les vannes V1, V2 et V3 restent fermées. Puis les première et seconde parois 21 et 11 sont écartées l'une de l'autre, jusqu'à ce qu'elles soient en position initiale, en remontant le piston 20 dans la chambre 10 dans le sens inverse de la flèche F1.

Il est alors possible de récupérer le lysat de cellules biologiques.

Dans le mode de réalisation des figures 7 et 8, cette récupération est obtenue en ouvrant les vannes V1 et V3, et en faisant circuler un liquide d'élution entre l'entrée 30 et la sortie fluidique 40 pour collecter le matériel biologique lysé. Puis le liquide de nettoyage comprenant le lysat est récupéré par la sortie fluidique 40.

La sortie fluidique 40 peut directement être reliée de manière fluidique à un appareil d'analyse.

Toutes ces opérations peuvent être faites dans un circuit fermé et étanche évitant toute contamination du lysat. En effet, il n'est pas nécessaire de retirer des billes du lysat, comme dans les procédés actuels. En outre, le matériel biologique récupéré ne risque pas d'être endommagé par des produits chimiques, cassé par des ultrasons, ou déstructuré par la chaleur.

Le cisaillement des cellules biologiques entre deux parois, dont une au moins est rugueuse, permet une lyse en quelques secondes, avec un dispositif simple, compact et peu onéreux.

La figure 9 illustre un septième mode de réalisation évitant de nettoyer le piston après chaque lyse.

Dans ce mode de réalisation, la chambre comporte, en outre, une membrane étanche 50 agencée entre la première paroi 21, l'entrée 30 et la sortie fluidique 40. La membrane 50 doit être suffisamment fine et souple pour épouser, en position de lyse, la rugosité de la paroi rugueuse, qui peut être portée par le piston et/ou par la chambre.

A titre d'exemple, on peut utiliser un film polymère ou en plastique d'épaisseur comprise entre 50 µm et quelques centaines de µm, par exemple 100 µm, la paroi rugueuse et microporeuse pouvant être un filtre en acier inoxydable.

Après la lyse, le piston peut être récupéré sans avoir à être nettoyé, et la chambre peut être soit nettoyée pour une prochaine lyse, soit éliminée.

Cette solution est envisageable grâce à la possibilité d'intégrer le dispositif selon l'invention dans un système de type « lab-on-a-chip » en anglais, ou « laboratoire sur puce ». La chambre est alors constituée dans la puce, par l'espace situé entre l'entrée fluidique, la sortie fluidique et la seconde paroi rugueuse.

Dans ce cas, l'utilisation d'une membrane et d'un piston rapportés permet d'effectuer la lyse sur une puce à usage unique, de récupérer le lysat, puis d'éliminer la puce. La membrane peut être lavée ou éliminée, et le piston est récupéré sans avoir à être lavé pour une utilisation ultérieure, sur un autre consommable,

Bien entendu, on comprend que le mot « piston » couvre, dans ce cas, toute pièce permettant d'appliquer un cisaillement mécanique des cellules contre la paroi de la chambre, cette pièce n'ayant pas nécessairement la forme classique d'un piston. C'est ainsi la membrane qui assure l'étanchéité, et non le piston qui peut alors avoir n'importe quelle forme.

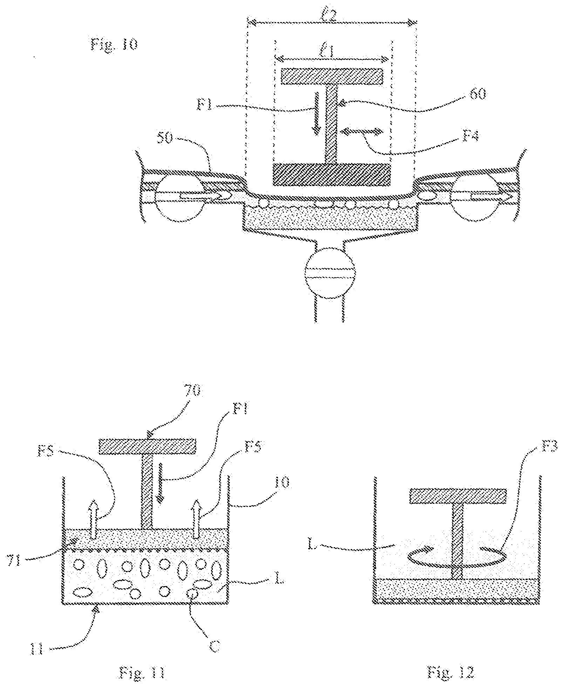

Il pourrait s'agir, par exemple, d'une spatule métallique appliquée en cisaillement par l'utilisateur, ou d'un tournevis automatique. Le terme « tournevis automatique » désigne un piston 60 présentant une partie supérieure et une partie inférieure, tel que, lorsque la partie supérieure décrit une translation, la partie inférieure du piston décrit un mouvement de rotation lorsqu'une résistance s'oppose à sa translation. La mise en œuvre d'un tel dispositif est illustrée à la figure 10 dans laquelle le piston 60 présente des dimensions (ici la largeur ℓ1, vue en coupe) inférieures aux mêmes dimensions (ici la largeur ℓ2, vue en coupe) de la chambre, de sorte que le cisaillement n'est pas obtenu nécessairement par rotation du piston 60, mais peut également être obtenu par translation, comme illustré par la flèche F4.

Les figures 11 et 12 illustrent un autre mode de réalisation dans lequel c'est le piston 70 qui présente une paroi 71 microporeuse. Ainsi, lorsqu'il est descendu en position de lyse contre la seconde paroi 11 (lisse ou rugueuse) de la chambre 10, le liquide L diffuse à travers la paroi microporeuse 71 du piston 70 selon le sens des flèches F5, et peut être évacué, par exemple en renversant la chambre 10 ou en aspirant le liquide L. Ce mode de réalisation peut également être combiné avec une chambre présentant une entrée et une sortie fluidique (non illustré).

Le dispositif et le procédé selon l'invention permettent d'obtenir une lyse très rapide d'espèces biologiques, sans nécessiter l'ajout de moyens complémentaires de lyse dans la chambre de lyse (bille, réactif chimique, apport de chaleur, ultrasons, etc.).

La mise en œuvre est simple et peut être réalisée manuellement ou automatiquement

En plus d'être plus rapide, le procédé selon l'invention est plus efficace que le procédé de référence (broyeur Precellys© (Bertin)).

L'efficacité de la lyse est déterminée indirectement par la quantité d'ADN libérée après la lyse. Cette quantité d'ADN est évaluée par « q-PCR ».

Les espèces, en contact avec une surface rugueuse, sont soumises à des forces de cisaillement et de pression à l'aide soit d'une surface non rugueuse, telle que la surface d'une spatule métallique ou d'un cône de pipette en plastique, soit à l'aide d'une seconde surface rugueuse (cas d'une lame de verre dépoli sur verre dépoli).

Les espèces sont broyées pendant une durée de 2 secondes afin de libérer au moins un des constituants cellulaires dans le milieu (ADN, ARN, protéine,...).

Par exemple, le procédé a été testé dans les configurations suivantes :
- verre dépoli (Ra = 0,425 µm) / cellules biologiques / verre dépoli (Ra = 0,425 µm) ;
- embout de pipette plastique (Ra non mesurable / cellules biologiques / Verre dépoli (Ra = 0,425 µm) ;
- spatule plate métallique (Ra ≤ 0,2 µm) / film polymère / cellules biologiques / verre dépoli (Ra = 0,425 µm) sur lequel on a appliqué un filtre polymère poreux, par exemple un filtre « Cyclopore Polycarbonate » de porosité 0,4 µm, commercialisé par Whatman (référence) et filmé ;
- PMMA dépoli par sablage (Ra = 0,92 µm / cellules biologiques / PMMA dépoli (Ra = 0,92 µm) ;
- embout de pipette plastique (Ra non mesurable) / cellules biologiques / PMMA dépoli (Ra = 0,92 µm) ;
- spatule plate métallique (Ra ≤ 0,2 µm) / film plastique / cellules biologiques / PMMA dépoli (Ra = 0,92 µm).

L'utilisation du film plastique est destinée à éviter la contamination des espèces lors de l'utilisation d'une spatule en acier.

Il est à noter que :
- deux types de verre dépoli (Ra = 0,425 µm - Ra = 1,98 µm), ont été testés,
- le procédé a été validé sur des espèces réputées difficiles à lyser, telles les spores Bg (*Bacillus globigii*), Bs (*Bacillus subtilis*),
- les lyses ont été réalisées à sec ou dans un volume de 0,5 µL à 5 pL de solution aqueuse,
- les lyses ont été réalisées sur un nombre de spores compris entre 100 et 10⁵ spores par lyse.

Chaque de paramètre de rugosité Ra a été déterminé de la façon suivante :
- mesure d'un profil brut à l'aide d'un profilomètre « Talysurf »,
- établissement d'un profil primaire en appliquant un filtrage de forme (dit « form removal ») au profil brut,
- établissement d'un profil de rugosité en appliquant un filtre « roughness filter » au profil primaire,
- détermination du paramètre Ra à partir dudit profil de rugosité.

Une première série d'essais a visé à comparer l'efficacité du procédé selon l'invention (deux déclinaisons : verre sur verre, et verre sur plastique) avec la méthode de référence par Precellys (Bertin). La quantité d'ADN libéré après la lyse des spores est évaluée par « q-PCR », à l'aide d'une machine « Stratagene » (Agilent) étalonnée conformément aux instructions du constructeur

Les résultats de la première déclinaison ont été les suivants :
1. Avec la « PCR » sur des spores lysées par Precellys (Bertin), on a obtenu un Ct (« Cycle Threshold » en anglais) égal à 31.
2. Le procédé selon l'invention a été appliqué à la même solution de spores dans la déclinaison verre sur verre, c'est-à-dire qu'on a lysé les spores entre deux lames de verre dépoli de Ra = 0,425 µm. Avec la « PCR » sur les spores lysées par le procédé selon l'invention, on a obtenu un Ct égal à 30, ce qui indique que la concentration en spores lysées est deux fois supérieure à celle de l'essai précédent.
3. Une « PCR » directe a été réalisée sur un témoin non lysé, et l'on a obtenu un Ct égal à 35,5 (la plupart des spores ne sont pas lysées dans la solution initiale).

Les résultats de la seconde déclinaison ont été les suivants :
1. Avec la « PCR » sur des spores lysées par Precellys (Bertin), on a obtenu un Ct égal à 30.
2. Le procédé selon l'invention a été appliqué à la même solution de spores dans la déclinaison verre sur cône de pipette en plastique, c'est-à-dire qu'on a lysé les spores entre une lame de verre de Ra = 0,425 µm et un cône de pipette en plastique. La « PCR » sur les spores lysées par le procédé selon l'invention a conduit à un Ct égal à 29 ce qui indique que la concentration en spores lysées est deux fois supérieure à l'essai précédent,
3. Avec une « PCR » directe réalisée sur un témoin non lysé, on a obtenu un Ct égal à 36 (la plupart des spores ne sont pas lysées dans la solution initiale).

Ces résultats montrent :
1. que le procédé selon l'invention conduit à une lyse des spores (Ct=30 ou 29 contre 35,5 ou 36 pour le témoin). Il y a au moins 5 Ct d'écart entre la solution lysée par le procédé selon l'invention et la solution non lysée, ce qui correspond à un ratio de 2⁵.
2. que le procédé est plus efficace que le procédé de lyse de référence : ΔCt=1 (30-31 et 29-30), c'est-à-dire que le procédé selon l'invention permet de récupérer deux fois plus de matériel.

Une seconde série d'essais a visé à contrôler la non-adsorption et la non-dégradation de l'ADN par les surfaces mises en jeu dans le procédé selon l'invention.

On a comparé :
1. Une « PCR » directe réalisée sur une solution d'ADN pur + spores, avec obtention d'un Ct égal à 31, à
2. Une « PCR » réalisée sur une solution d'ADN pur + spores soumise à une lyse réalisée conformément au procédé selon l'invention dans la déclinaison verre sur cône de pipette en plastique, avec obtention d'un Ct égal à 31.

Ces résultats montrent que la « PCR » sur la solution d'ADN pur + spores donne les mêmes résultats que lorsque la solution a subi le procédé selon l'invention, Par conséquent :
1. Le procédé ne détruit pas l'ADN,
2. Il n'y a pas d'adsorption de l'ADN par les surfaces.
3. La récupération des échantillons est efficace (pas de pertes car même Ct).

## Revendications

1. Dispositif de lyse mécanique (1) d'espèces biologiques (C) présentes dans un fluide comprenant une première paroi (21, 71) et une seconde paroi (11) montées mobiles l'une par rapport à l'autre, entre une position initiale dans laquelle les première et seconde parois sont écartées l'une de l'autre, et une position de lyse dans laquelle la première paroi est en appui contre la seconde paroi, les première et seconde parois étant également montées mobiles en cisaillement l'une par rapport à l'autre dans la position de lyse, **caractérisé en ce que** l'une au moins des première et seconde parois a une surface d'appui rugueuse contre l'autre paroi et présente un paramètre de rugosité de surface Ra moyen compris entre 0,2 µm et 10 µm, et préférentiellement entre 0,2 µm et 3 µm.

2. Dispositif de lyse mécanique (1) selon la revendication 1, dans lequel l'une des première paroi (21, 71) et seconde paroi (11) est une paroi microporeuse (11, 71), c'est-à-dire poreuse aux liquides et non poreuse aux espèces biologiques (C) devant être lysées.

3. Dispositif de lyse mécanique (1) selon la revendication 2, dans lequel la paroi microporeuse (11,71) présente un diamètre moyen de pores inférieur à 1 µm, voire inférieur à 0,2 µm.

4. Dispositif de lyse mécanique (1) selon l'une quelconque des revendications 2 ou 3, dans lequel la paroi microporeuse (11) est rugueuse.

5. Dispositif de lyse mécanique (1) selon la revendication 4, dans lequel la paroi microporeuse rugueuse (11) est constituée d'une couche de matériau microporeux (11) ayant une surface plane sur laquelle est positionnée une couche rugueuse (14) destinée à être en appui contre l'autre paroi (21) en position de lyse.

6. Dispositif de lyse mécanique (1) selon la revendication 4, dans lequel la paroi microporeuse rugueuse (11) est constituée d'une couche de matériau microporeux (11) ayant une surface rugueuse (15).

7. Dispositif de lyse mécanique (1) selon la revendication 4, dans lequel la paroi microporeuse rugueuse (11) est constituée d'une couche de matériau macroporeux (16) ayant une surface rugueuse (17) sur laquelle est positionnée une couche microporeuse (18) destinée à être en appui contre l'autre paroi (21) en position de lyse, la couche microporeuse (18) étant suffisamment souple et fine pour épouser la rugosité de la surface de la couche de matériau macroporeux (16) en position de lyse.

8. Dispositif de lyse mécanique (1) selon l'une quelconque des revendications 2 ou 3, dans lequel la paroi microporeuse (11) est non rugueuse et présente un paramètre de rugosité de surface Ra moyen inférieur à 0,1 µm, alors que la paroi non microporeuse (21) est rugueuse et présente un paramètre de rugosité de surface Ra moyen compris entre 0,2 µm et 10 µm, préférentiellement entre 0,2 µm et 3 µm.

9. Dispositif de lyse mécanique (1) selon l'une quelconque des revendications 4 à 7, dans lequel la première paroi (21) et la seconde paroi (11) sont rugueuses.

10. Dispositif de lyse mécanique (1) selon l'une quelconque des revendications 1 à 9, **caractérisé en ce qu'**il comprend, en outre, une chambre (10) et un piston (20) monté mobile dans la chambre entre la position initiale et la position de lyse, la première paroi (21) étant montée sur le piston mobile et la seconde paroi (11) étant agencée en regard de la première paroi.

11. Dispositif de lyse mécanique (1) selon la revendication 10 lorsqu'elle dépend de l'une des revendications 2 à 9, dans lequel la seconde paroi (11) est microporeuse, et dans lequel la chambre (10) comporte, en outre, une sortie fluidique (12) agencée à l'opposé de la première paroi (21) par rapport à la seconde paroi microporeuse (11).

12. Dispositif de lyse mécanique (1) selon l'une quelconque des revendications 2 à 11, dans lequel la chambre (10) comporte, en outre, une entrée fluidique (30) et une autre sortie fluidique (40).

13. Dispositif de lyse mécanique (1) selon la revendication 12 lorsqu'elle dépend de la revendication 11, dans lequel la chambre (10) comporte, en outre, une membrane étanche (50) agencée entre la première paroi (21), l'entrée fluidique (30) et la sortie fluidique (40), la membrane étant suffisamment fine et souple pour épouser la rugosité de l'une au moins des première et seconde parois (21 et 11) en position de lyse.

14. Procédé de lyse mécanique d'espèces biologiques (C), **caractérisé en ce qu'**il comprend les étapes suivantes :
A) Fourniture d'un dispositif (1) selon l'une quelconque des revendications 1 à 13, de telle sorte que les première et seconde parois (21 et 11) soient en position initiale ;
B) Placer les espèces biologiques à lyser entre les première et seconde parois ;
C) Amener les première et seconde parois en position de lyse ;
D) Générer un cisaillement entre les première et seconde parois en position de lyse, pour provoquer la lyse mécanique des espèces biologiques et obtenir un lysat ;
E) Écarter les première et seconde parois jusqu'à ce qu'elles soient en position initiale ; et
F) Récupérer le lysat des espèces biologiques.

15. Procédé de lyse mécanique d'espèces biologiques (C) selon la revendication précédente, dans lequel :
- l'étape A) consiste à fournir un dispositif (1) selon la revendication 10,
- l'étape C) consiste à faire coulisser le piston (20) dans la chambre (10) jusqu'à ce que les première et seconde parois (21 et 11) soient en position de lyse ; et
- l'étape D) consiste à faire tourner le piston sur lui-même de telle sorte que les première et seconde parois restent en contact l'une avec l'autre.

16. Procédé de lyse mécanique d'espèces biologiques (C) selon la revendication précédente, dans lequel :
- l'étape A) consiste à fournir un dispositif (1) selon la revendication 11 ;
- l'étape B) consiste à placer un liquide comportant les espèces biologiques à lyser entre les première et seconde parois (21 et 11) ;
- l'étape C) consiste à faire coulisser le piston dans la chambre jusqu'à ce que le liquide ait été filtré à travers la paroi microporeuse (11), que seules les espèces biologiques à lyser restent sur la paroi microporeuse (11) et que les première et seconde parois soient en position de lyse ; et
- l'étape D) consiste à faire tourner le piston (20) sur lui-même de telle sorte que les première et seconde parois restent en contact l'une avec l'autre.

17. Procédé de lyse mécanique d'espèces biologiques (C) selon la revendication précédente, dans lequel :
- l'étape A) consiste à fournir un dispositif (1) selon la revendication 12 ;
- l'étape B) consiste à faire entrer un liquide comportant les espèces biologiques à lyser par l'entrée fluidique (30), entre les première et seconde parois (21 et 11) ;
- l'étape C) consiste à faire coulisser le piston (20) dans la chambre (10) jusqu'à ce que les première et seconde parois soient en position de lyse ;
- l'étape D) consiste à faire tourner le piston sur lui-même de telle sorte que les première et seconde parois restent en contact l'une avec l'autre ; et
- l'étape F) consiste à faire circuler un liquide d'élution entre l'entrée fluidique (30) et la sortie fluidique (40) pour récupérer le lysat par la sortie fluidique.

18. Procédé de lyse mécanique d'espèces biologiques (C) selon la revendication précédente, dans lequel :
- l'étape A) consiste à fournir un dispositif (1) selon la revendication 13,
- l'étape B) consiste à faire entrer un liquide comportant les espèces biologiques à lyser par l'entrée fluidique (30), entre la membrane étanche (50) et la seconde paroi (11) ;
- l'étape C) consiste à faire coulisser le piston (20) dans la chambre (10) jusqu'à ce que les première et seconde parois (21 et 11) soient en position de lyse par l'intermédiaire de la membrane étanche ;
- l'étape D) consiste à faire tourner le piston sur lui-même de telle sorte que les première et seconde parois restent en contact l'une avec l'autre par l'intermédiaire de la membrane étanche ; et
- l'étape F) consiste à faire circuler un liquide d'élution entre l'entrée fluidique (30) et la sortie fluidique (40) pour récupérer le lysat par la sortie fluidique.

## Patentansprüche

1. Vorrichtung zur mechanischen Lyse (1) biologischer Spezies (C), die in einem Fluid vorhanden sind, umfassend eine erste Wand (21, 71) und eine zweite Wand (11), die in Bezug zueinander zwischen einer Ausgangsposition, in der die erste und zweite Wand voneinander entfernt sind, und einer Lyseposition beweglich montiert sind, in der die erste Wand an der zweiten Wand anliegend ist, wobei die erste und zweite Wand in der Lyseposition auch in Scherung in Bezug zueinander beweglich montiert sind, **dadurch gekennzeichnet, dass** die mindestens eine der ersten und zweiten Wand eine raue Anlagefläche an der anderen Wand aufweist, und einen mittleren Oberflächenrauheitsparameter Ra vorweist, der zwischen 0,2 µm und 10 µm, und vorzugsweise zwischen 0,2 µm und 3 µm enthalten ist.

2. Vorrichtung zur mechanischen Lyse (1) nach Anspruch 1, wobei die eine der ersten Wand (21, 71) und zweiten Wand (11) eine mikroporöse Wand (11, 71) ist, das heißt porös für Flüssigkeiten und nicht porös für biologische Spezies (C), die zu lysieren sind.

3. Vorrichtung zur mechanischen Lyse (1) nach Anspruch 2, wobei die mikroporöse Wand (11, 71) einen mittleren Porendurchmesser kleiner als 1 µm, oder gar kleiner als 0,2 µm vorweist.

4. Vorrichtung zur mechanischen Lyse (1) nach einem der Ansprüche 2 oder 3, wobei die mikroporöse Wand (11) rau ist.

5. Vorrichtung zur mechanischen Lyse (1) nach Anspruch 4, wobei die raue mikroporöse Wand (11) aus einer mikroporösen Materialschicht (11) gebildet ist, die eine ebene Oberfläche aufweist, auf der eine raue Schicht (14) positioniert ist, die dazu bestimmt ist, sich in der Lyseposition an der anderen Wand (21) anzulegen.

6. Vorrichtung zur mechanischen Lyse (1) nach Anspruch 4, wobei die raue mikroporöse Wand (11) aus einer mikroporösen Materialschicht (11) gebildet ist, die eine raue Oberfläche (15) aufweist.

7. Vorrichtung zur mechanischen Lyse (1) nach Anspruch 4, wobei die raue mikroporöse Wand (11) aus einer makroporösen Materialschicht (16) gebildet ist, die eine raue Oberfläche (17) aufweist, auf der eine mikroporöse Schicht (18) positioniert ist, die dazu bestimmt ist, sich in der Lyseposition an der anderen Wand (21) anzulegen, wobei die mikroporöse Schicht (18) ausreichend flexibel und fein ist, um die Rauheit der Oberfläche der makroporösen Materialschicht (16) in der Lyseposition anzunehmen.

8. Vorrichtung zur mechanischen Lyse (1) nach einem der Ansprüche 2 oder 3, wobei die mikroporöse Wand (11) nicht rau ist und einen mittleren Oberflächenrauheitsparameter Ra kleiner als 0,1 µm vorweist, während die nicht mikroporöse Wand (21) rau ist und einen mittleren Oberflächenrauheitsparameter Ra vorweist, der zwischen 0,2 µm und 10 µm, vorzugsweise zwischen 0,2 µm und 3 µm enthalten ist.

9. Vorrichtung zur mechanischen Lyse (1) nach einem der Ansprüche 4 bis 7, wobei die erste Wand (21) und die zweite Wand (11) rau sind.

10. Vorrichtung zur mechanischen Lyse (1) nach einem der Ansprüche 1 bis 9, **dadurch gekennzeichnet, dass** sie weiter eine Kammer (10) und einen Kolben (20) umfasst, der in der Kammer zwischen der Ausgangsposition und der Lyseposition beweglich montiert ist, wobei die erste Wand (21) auf dem beweglichen Kolben montiert ist und die zweite Wand (11) gegenüber der ersten Wand angeordnet ist.

11. Vorrichtung zur mechanischen Lyse (1) nach Anspruch 10, wenn er von einem der Ansprüche 2 bis 9 abhängig ist, wobei die zweite Wand (11) mikroporös ist, und wobei die Kammer (10) weiter einen Fluidausgang (12) umfasst, der gegenüber der ersten Wand (21) in Bezug auf die zweite mikroporöse Wand (11) angeordnet ist.

12. Vorrichtung zur mechanischen Lyse (1) nach einem der Ansprüche 2 bis 11, wobei die Kammer (10) weiter einen Fluideingang (30) und einen weiteren Fluidausgang (40) umfasst.

13. Vorrichtung zur mechanischen Lyse (1) nach Anspruch 12, wenn er von Anspruch 11 abhängig ist, wobei die Kammer (10) weiter eine dichte Membran (50) umfasst, die zwischen der ersten Wand (21), dem Fluideingang (30) und dem Fluidausgang (40) angeordnet ist, wobei die Membran ausreichend fein und flexibel ist, um die Rauheit der mindestens einen der ersten und zweiten Wand (21 und 11) in der Lyseposition anzunehmen.

14. Verfahren zur mechanischen Lyse biologischer Spezies (C), **dadurch gekennzeichnet, dass** es die folgenden Schritte umfasst:
A) Bereitstellen einer Vorrichtung (1) nach einem der Ansprüche 1 bis 13, derart, dass die erste und zweite Wand (21 und 11) in der Ausgangsposition sind;
B) Platzieren der zu lysierenden biologischen Spezies zwischen der ersten und zweiten Wand;
C) Herbeiführen der ersten und zweiten Wand in die Lyseposition;
D) Generieren einer Scherung zwischen der ersten und zweiten Wand in der Lyseposition, um die mechanische Lyse der biologischen Spezies zu bewirken und ein Lysat zu erhalten;
E) Trennen der ersten und zweiten Wand, bis sie in der Ausgangsposition sind; und
F) Einsammeln des Lysats der biologischen Spezies.

15. Verfahren zur mechanischen Lyse biologischer Spezies (C) nach dem vorstehenden Anspruch, wobei:
- der Schritt A) darin besteht, eine Vorrichtung (1) nach Anspruch 10 bereitzustellen,
- der Schritt C) darin besteht, den Kolben (20) in die Kammer (10) gleiten zu lassen, bis die erste und zweite Wand (21 und 11) in der Lyseposition sind; und
- der Schritt D) darin besteht, den Kolben derart um sich selbst drehen zu lassen, dass die erste und zweite Wand in Kontakt miteinander bleiben.

16. Verfahren zur mechanischen Lyse biologischer Spezies (C) nach dem vorstehenden Anspruch, wobei:
- der Schritt A) darin besteht, eine Vorrichtung (1) nach Anspruch 11 bereitzustellen,
- der Schritt B) darin besteht, eine Flüssigkeit, die die zu lysierenden biologischen Spezies umfasst, zwischen der ersten und zweiten Wand (21 und 11) zu platzieren;
- der Schritt C) darin besteht, den Kolben in die Kammer gleiten zu lassen, bis die Flüssigkeit durch die mikroporöse Wand (11) hindurch gefiltert worden ist, dass nur die zu lysierenden biologischen Spezies an der mikroporösen Wand (11) bleiben und die erste und zweite Wand in der Lyseposition sind; und
- der Schritt D) darin besteht, den Kolben (20) derart um sich selbst drehen zu lassen, dass die erste und zweite Wand in Kontakt miteinander bleiben.

17. Verfahren zur mechanischen Lyse biologischer Spezies (C) nach dem vorstehenden Anspruch, wobei:
- der Schritt A) darin besteht, eine Vorrichtung (1) nach Anspruch 12 bereitzustellen,
- der Schritt B) darin besteht, durch den Fluideingang (30) eine Flüssigkeit, die die zu lysierenden biologischen Spezies umfasst, zwischen die erste und zweite Wand (21 und 11) eintreten zu lassen;
- der Schritt C) darin besteht, den Kolben (20) in die Kammer (10) gleiten zu lassen, bis die erste und zweite Wand in der Lyseposition sind; und
- der Schritt D) darin besteht, den Kolben derart um sich selbst drehen zu lassen, dass die erste und zweite Wand in Kontakt miteinander bleiben; und
- der Schritt F) darin besteht, eine Eluierungsflüssigkeit zwischen dem Fluideingang (30) und dem Fluidausgang (40) zirkulieren zu lassen, um das Lysat über den Fluidausgang einzusammeln.

18. Verfahren zur mechanischen Lyse biologischer Spezies (C) nach dem vorstehenden Anspruch, wobei:
- der Schritt A) darin besteht, eine Vorrichtung (1) nach Anspruch 13 bereitzustellen,
- der Schritt B) darin besteht, durch den Fluideingang (30) eine Flüssigkeit, die die zu lysierenden biologischen Spezies umfasst, zwischen die dichte Membran (50) und die zweite Wand (11) eintreten zu lassen;
- der Schritt C) darin besteht, den Kolben (20) in die Kammer (10) gleiten zu lassen, bis die erste und zweite Wand (21 und 11) über die dichte Membran in der Lyseposition sind;
- der Schritt D) darin besteht, den Kolben derart um sich selbst drehen zu lassen, dass die erste und zweite Wand über die dichte Membran in Kontakt miteinander bleiben; und
- der Schritt F) darin besteht, eine Eluierungsflüssigkeit zwischen dem Fluideingang (30) und dem Fluidausgang (40) zirkulieren zu lassen, um das Lysat über den Fluidausgang einzusammeln.

## Claims

1. Device (1) for the mechanical lysis of biological species (C) contained in a fluid, comprising a first wall (21, 71) and a second wall (11) mounted movable relative to one another between an initial position in which the first and second walls are drawn apart and a lysis position in which the first wall presses against the second wall, the first and second walls also being mounted movable under shear relative to one another in the lysis position, **characterized in that** at least one of the first and second walls has a rough bearing surface against the other wall and has an average surface roughness parameter Ra of between 0.2 µm and 10 µm, preferably between 0.2 µm and 3 µm.

2. Mechanical lysis device (1) according to claim 1, wherein one of the first (21, 71) and second (11) walls is a microporous wall (11, 71) i.e. porous to liquids and non-porous to biological species (C) to be lysed.

3. The mechanical lysis device (1) according to claim 2, wherein the microporous wall (11, 71) has a mean pore diameter of less than 1 µm, even less than 0.2 µm.

4. Mechanical lysis device (1) according to any of claims 2 or 3, wherein the microporous wall (11) is rough.

5. Mechanical lysis device (1) according to claim 4, wherein the rough microporous wall (11) is formed of a layer of microporous material (11) having a planar surface on which a rough layer (14) is positioned intended to press against the other wall (21) in lysis position.

6. Mechanical lysis device (1) according to claim 4, wherein the rough microporous wall (11) is formed of a layer of microporous material (11) having a rough surface (15).

7. Mechanical lysis device (1) according to claim 4, wherein the rough microporous wall (11) is formed of a layer of macroporous material (16) having a rough surface (17) on which a macroporous layer (18) is positioned intended to press against the other wall (21) in lysis position, the microporous layer (18) being sufficiently thin and flexible to follow the contour of the surface roughness of the layer in macroporous material (16) in lysis position.

8. Mechanical lysis device (1) according to any of claims 2 or 3, wherein the microporous wall (11) is not rough and has an average surface roughness parameter Ra of less than 0.1 µm, whereas the non-microporous wall (21) is rough and has an average surface roughness parameter Ra of between 0.2 µm and 10 µm, preferably between 0.2 µm and 3 µm.

9. Mechanical lysis device (1) according to any of claims 4 to 7, wherein the first wall (21) and the second wall (11) are rough.

10. The mechanical lysis device (1) according to any of claims 1 to 9, **characterized in that** it also comprises a chamber (10) and a piston (20) mounted mobile in the chamber between the initial position and the lysis position, the first wall (21) being mounted on the mobile piston and the second wall (11) being arranged facing the first wall.

11. Mechanical lysis device (1) according to claim 10 when it is dependent on one of claims 2 to 9, wherein the second wall (11) is microporous, and wherein the chamber (10) also comprises a fluid outlet (12) arranged opposite the first wall (21) relative to the second microporous wall (11).

12. Mechanical lysis device (1) according to any of claims 2 to 11, wherein the chamber (10) also comprises a fluid inlet (30) and another fluid outlet (40).

13. The mechanical lysis device (1) according to claim 12 when it is dependent on claim 11, wherein the chamber (10) also comprises an impervious membrane (50) arranged between the first wall (21), the fluid inlet (30) and fluid outlet (40), the membrane being sufficiently thin and flexible to follow the contour of the roughness of at least one among the first and second walls (21 and 11) in lysis position.

14. Method for the mechanical lysis of biological species (C), **characterized in that** it comprises the following steps:
A) Providing a device (1) according to any of claims 1 to 13 so that the first and second walls (21 and 11) lie in initial position;
B) Placing the biological species to be lysed between the first and second walls;
C) Bringing the first and second walls to lysis position;
D) Generating shear between the first and second walls in lysis position to cause mechanical lysis of the biological species and to obtain a lysate;
E) Drawing the first and second walls apart until they reach the initial position; and
F) Recovering the lysate of the biological species.

15. Method for mechanical lysis of biological species (C) according to the preceding claim, wherein:
- at step A) a device (1) according to claim 10 is provided;
- at step C) the piston (20) is caused to slide inside the chamber (10) until the first and second walls (21 and 11) are in lysis position; and
- at step D) the piston is rotated so that the first and second walls remain in contact with each other.

16. Method for mechanical lysis of biological species (C) according to the preceding claim, wherein:
- at step A) a device (1) according to claim 11 is provided;
- at step B) a liquid containing the biological species to be lysed is placed between the first and second walls (21 and 11);
- at step C) the piston is caused to slide inside the chamber until the liquid has been filtered through the microporous wall (11), solely the biological species to be lysed are left remaining on the microporous wall (11) and the first and second walls are in lysis position; and
- at step D) the piston (20) is rotated so that the first and second walls remain in contact with each other.

17. Method for mechanical lysis of biological species (C) according to the preceding claim, wherein:
- at step A) a device (1) according to claim 12 is provided;
- at step B) the liquid containing the biological species to be lysed is caused to enter through the fluid inlet (30) between the first and second walls (21 and 11);
- at step C) the piston (20) is caused to slide inside the chamber (10) until the first and second walls are in lysis position;
- at step D) the piston is rotated so that the first and second walls remain in contact with each other; and
- at step F) an eluting liquid is circulated between the fluid inlet (30) and the fluid outlet (40) to recover the lysate through the fluid outlet.

18. Method for mechanical lysis of biological species (C) according to the preceding claim, wherein:
- at step A) a device (1) according to claim 13 is provided;
- at step B) a liquid containing the biological species to be lysed is caused to enter through the fluid inlet (30) between the impervious membrane (50) and the second wall (11);
- at step C) the piston (20) is caused to slide inside the chamber (10) until the first and second walls (21 and 11) are in lysis position via the impervious membrane;
- at step D) the piston is rotated so that the first and second walls remain in contact with each other via the impervious membrane; and
- at step F) an eluting liquid is circulated between the fluid inlet (30) and fluid outlet (40) to recover the lysate through the fluid outlet.
